# EUROPEAN PATENT SPECIFICATION

(11) **EP 4 378 970 B1**
(45) Date of publication and mention of the grant of the patent: **06.05.2026**
(21) Application number: 22860070.6
(22) Date of filing: 08.07.2022
(51) Int. Cl.: C08F 222/10, C08F 220/20, C08F 2/44, C08K 3/36, C08K 3/40, B33Y 70/10, A61L 27/44, A61K 6/78, A61K 6/887, C08F 290/06, B33Y 10/00, C08F 2/50, A61K 6/64, A61K 6/17, A61K 6/62, A61K 6/76, A61K 6/77

(54) **PHOTOTHERMAL-CURING RESIN COMPOSITION, AND PREPARATION METHOD THEREFOR AND USE THEREOF**
PHOTOTHERMISCH HÄRTBARE HARZZUSAMMENSETZUNG UND HERSTELLUNGSVERFAHREN DAFÜR UND VERWENDUNG DAVON
COMPOSITION DE RÉSINE À DURCISSEMENT PHOTOTHERMIQUE, PROCÉDÉ DE PRÉPARATION ASSOCIÉ ET UTILISATION CORRESPONDANTE

(30) Priority: 26.08.2021 CN 202110985937
(43) Date of publication of application: 05.06.2024
(73) Proprietor: Aidite (Qinhuangdao) Technology Co., Ltd., Qinhuangdao Hebei 066004 (CN)
(72) Inventor: ZHANG, Jiaxin, Qinhuangdao, Hebei 066004 (CN); LI, Hongwen, Qinhuangdao, Hebei 066004 (CN); DUAN, Guangyuan, Qinhuangdao, Hebei 066004 (CN); XING, Linlin, Qinhuangdao, Hebei 066004 (CN)
(74) Representative: Larsen & Birkeholm A/S
(86) International application number: PCT/CN2022/104492
(87) International publication number: WO 2023/024724

(56) References cited:
- WO-A1-2020/209505
- WO-A1-2021/030512
- CN-A- 1 212 865
- CN-A- 106 947 034
- CN-A- 108 014 021
- CN-A- 108 912 287
- CN-A- 113 717 330
- US-A- 4 264 489
- US-A- 5 977 199
- US-A- 6 133 339
- US-A1- 2012 021 383
- US-A1- 2020 199 267
- US-A1- 2020 253 838

## Description

### Technical Field

The present disclosure belongs to the technical field of resin materials, and specifically relates to a photothermal-curing resin composition, and preparation method therefor and use thereof.

### Background Art

A temporary crown and bridge is a type of temporary tooth crown or bridge body worn by a patient before finally wearing the tooth crown, which is used in a field of oral implantology and restoration. The conventional method to make the temporary crown and bridge is mainly to manually mix denture powder and denture water to mold. The molding is generally divided into intraoral molding or extraoral molding. The residual methacrylic acid resin has a very irritating and pungent odor, thus providing the patient with a pretty poor treatment experience. Currently, some processing plants use cutting technology to make PMMA temporary crown and bridge. The PMMA realizes digital processing, which increases the processing efficiency, but this technology has a very low material utilization rate, so that the material is wasted seriously. Additionally, the cutting process generates dust, which is less environmentally friendly.

3D printing is a type of additive manufacturing process, which can complete a processing of elaborate or hollow workpiece that is difficult to be made by cutting and is especially suitable for personalized data production, such as the temporary crown and bridge. The material utilization rate of the additive manufacturing is very high, which can save costs. Therefore, the use of 3D printing technology in the dental department is a key to realize a digitalization of the dental department. The 3D printing material used in the dental department is generally the photosensitive resin, which is cured and accumulated layer by layer via laser surface molding or spot molding on a 3D printing device with a certain emission wavelength, such that finally the desired printed workpiece is formed.

In the method of 3D printing, it needs only two steps, namely, obtaining data and wearing the temporary crown, to make the temporary crown and bridge, where the operation is simple and therefore the operation time can be greatly reduced. With the rapid development of additive manufacturing technology, the temporary crown and bridge by 3D printing brings a new direction. However, the resin used for the 3D printing of the temporary crown and bridge not only needs a higher biocompatibility for the cured material, but also needs a better mechanical property. The performance of the temporary crown and bridge obtained by the 3D printing of the simple photo-curing system cannot satisfy the use of the temporary tooth crown. The completion of the photo-curing parts in the system after printed and molded is low and the overall performance is poor, and there will be the problem of stress shrinkage.

The Chinese patent CN106947034A discloses a thermal post curing 3D printing photosensitive resin, a preparation method and application thereof. The photosensitive resin includes a prepolymer, diluent, photoinitiator, and thermal initiator, wherein a decomposition temperature of the thermal initiator at the half-life time of one hour is larger than or equal to 100° C. The weight parts of each of the components are as follows: 50~70 parts of the prepolymer, 30~50 parts of the diluent, 1~5 parts of the photoinitiator, and 0.5~5 parts of the thermal initiator. After molded by the photo-curing 3D printing, the post-curing method of the 3D printing photosensitive resin obtained by this invention can be selected from an oven or other stable constant-temperature heating equipment for thermal post curing. Therefore, the 3D printing photosensitive resin of thermal post curing is obtained, which extends the use in opaque samples, samples with complex shapes, ultra-thick samples, and the colored samples, etc., and also provides the samples with better mechanical property. However, the curing after printing and molding in the above prior art is only thermal curing, without further photo curing. The photo curing parts of this resin system has a low degree of completion and poor overall performance, and the resin compositions do not contain inorganic fillers, so that the resin composition ultimately obtained has stress shrinkage and low hardness.

The Chinese patent CN110128773A discloses a photo/thermal double curing 3D printing method and product thereof, including the following steps: mixing raw materials for photo-curing 3D printing, and then carrying out the thermal curing to obtain the 3D printed product, wherein the raw materials include: polyurethane acrylate, diluent, initiator, epoxy resin, and thermal curing agent, wherein the polyurethane acrylate is obtained by end capping by acrylate with hydroxyl groups after isocyanate reacts with polyols. The polyols are polyester polyols or polyether glycols with a molecular weight not less than 1000. The method of this invention avoids a phenomenon that the thermal curing of the epoxy resin leads the polymerization products to be brittle, which realizes the basic toughness of epoxy resin double-curing products, so as to meet the application in industrial manufacturing. The Chinese patent CN108948280A discloses a photo/thermal double curing 3D printing resin composition, by weight parts, including the following components: 66 ~ 86 parts of free radical type photosensitive resins, 9 ~ 29 parts of thermal curing epoxy resins, 1 ~ 4 parts of free radical type photoinitiator, and 1 ~ 3 parts of latent type thermal curing agents. The 3D printing resin composition obtained by this invention needs to be irradiated by UV with a wavelength of 365~500nm first, so that the acrylic resin therein carries out the polymerization induced by free radicals under the action of the photoinitiator. After molding, it is kept at 80~120°C for thermal preservation of 1~2h, so that the epoxy resin therein carries out the post-curing under the action of the latent type thermal curing agent. It can both accelerate a speed of photo curing and reduce a volume shrinkage rate of products. Additionally, it can enhance the physical and mechanical properties of the products by post thermal curing. The above two prior arts limit the synthesis method of the polyurethane acrylate and the molecular weight of the polyol, but the thermal curing system adopted by the two prior arts is mainly the epoxy curing system, wherein the epoxy resin system is brittle, high cost and may have compatibility problems with the acrylic resin system. Additionally, the added isobornyl ester has a larger odor, so that the environmental protection property is poor; and the resin compositions provided by the two prior arts do not add the inorganic component fillers, so that the abrasion resistance and strength are difficult to meet the actual demands. US 2012/021383 A1 discloses dental restorative materials such as crowns or bridges comprising a photothermal-curing resin composition and its preparation method, wherein the resin composition comprises the following components: a prepolymer, a monomer, a photoinitiator, a thermal initiator, fumed silica and a glass powder.

Therefore, it is an urgent technical problem to be addressed in this field to develop a resin composition with a high curing reaction completion rate, a higher hardness, and an abrasion resistance.

### Summary

In view of deficiencies of the prior art, the objective of the present disclosure is to provide a photothermal-curing resin composition, and preparation method therefor and use thereof. The resin composition includes a specific number of a prepolymer, monomer, photoinitiator, thermal initiator, fumed silica, and glass powder. By combining and matching the above substances, the resin composition obtained has a high curing reaction completion rate, and has a higher hardness and abrasion resistance, which can satisfy the demands of the resin material for a temporary crown and bridge.

To achieve this purpose, the present disclosure adopts the following technical solutions.

In a first aspect, the present disclosure provides a photothermal-curing resin composition, wherein the resin composition includes the following components by weight parts:

| | |
|---|---|
| a prepolymer | 30 weight parts; |
| a monomer | 5~50 weight parts; |
| a photoinitiator | 0.01~5 weight parts; |
| a thermal initiator | 0.01~5 weight parts; |
| fumed silica | 0.01~40 weight parts; and |

a glass powder 0.01~40 weight parts, wherein the prepolymer comprises any one or a combination of at least two of polyurethane acrylate, urethane dimethacrylate, bisphenol A-dimethacrylate glycidyl ester, ethoxybisphenol A dimethacrylate, or bisphenol A glycerol dimethacrylate, the monomer comprises any one or a combination of at least two of triethylene glycol dimethacrylate, hydroxyethyl methacrylate, or hydroxypropyl methacrylate, and the glass powder comprises glass powder with a particle size of 0.6-0.8 µm and glass powder with a particle size of 0.3-0.5 µm.

In the present disclosure, the monomer is in preferably 10 weight parts, 15 weight parts, 20 weight parts, 25 weight parts, 30 weight parts, 35 weight parts, 40 weight parts, or 45 weight parts.

In the present disclosure, the photoinitiator is in preferably 0.05 weight parts, 0.1 weight parts, 0.5 weight parts, 1 weight parts, 1.5 weight parts, 2 weight parts, 2.5 weight parts, 3 weight parts, 3.5 weight parts, 4 weight parts or 4.5 weight parts.

In the present disclosure, the thermal initiator is in preferably 0.05 weight parts, 0.1 weight parts, 0.5 weight parts, 1 weight parts, 1.5 weight parts, 2 weight parts, 2.5 weight parts, 3 weight parts, 3.5 weight parts, 4 weight parts or 4.5 weight parts.

In the present disclosure, the fumed silica is in preferably 0.05 weight parts, 1 weight parts, 5 weight parts, 10 weight parts, 15 weight parts, 20 weight parts, 25 weight parts, 30 weight parts, or 35 weight parts.

In the present disclosure, the glass powder is in preferably 0.05 weight parts, 1 weight parts, 5 weight parts, 10 weight parts, 15 weight parts, 20 weight parts, 25 weight parts, 30 weight parts, or 35 weight parts.

Most of the resin compositions for the temporary crown and bridge by 3D printing provided by the prior art first take unsaturated bonds in acrylic esters and the photoinitiator as photo-curing components, and are triggered to be cured by the UV light of the 3D printer to obtain the temporary crown and bridge of an initial shape. Then, the epoxy resin and the corresponding curing agent are used as the thermal curing component to make the temporary crown and bridge of the initial shape to be thermally cured in a constant temperature thermal curing box, so that the obtained temporary crown and bridge has a certain mechanical property. Mainly, the problem in the prior method is that the photoinduced curing reaction is insufficient when the light reaction is carried out in a 3D printer. After curing, a large portion of the monomer is still not photoinitiated, so there is a lot of monomer residue in the system. Even if the epoxy resin and its curing agent are thermally cured in a light box at a later stage, the photoinitiation system is also difficult to react in a thermal environment, which cannot compensate the problem of insufficient photo curing, so that the final obtained temporary crown and bridge cannot be used due to a larger amount of the monomer residues therein. Additionally, the epoxy resin is expensive, and the compatibility between the epoxy resin and the curing agent system thereof and the acrylic ester system is poor. Due to a lack of inorganic fillers, the abrasion resistance and hardness of the temporary crown and bridge obtained are limited, so that the scope of application is limited.

The photothermal-curing resin composition provided by the present disclosure includes the specific parts of the prepolymer, monomer, photoinitiator, thermal initiator, fumed silica, and glass powder. The specific parts of the photoinitiator and thermal initiator are added to the system, and the system is initiated by utilizing the photoinitiator and thermal initiator, thus allowing for a fuller cure. A completion rate of the curing reaction is improved, so that the residual amount of the monomer in the finally obtained resin composition can meet the standard requirements of the temporary crown and bridge. In the present disclosure, the system is further added specific number of fumed silica and glass powder containing barium as fillers to enhance the hardness, strength, and abrasion resistance of the material, so that the resin composition obtained has a higher hardness and abrasion resistance after 3D printed into the temporary crown and bridge, which fully satisfies the requirements of an oral restorative material or oral implant material.

The prepolymer includes any one or a combination of at least two of the polyurethane acrylate, urethane dimethacrylate, bisphenol A-dimethacrylate glycidyl ester, ethoxybisphenol A dimethacrylate, or bisphenol A glycerol dimethacrylate.

The monomer includes any one or a combination of at least two of triethylene glycol dimethacrylate, hydroxyethyl methacrylate, or hydroxypropyl methacrylate.

As a preferred technical solution of the present disclosure, the monomer and prepolymer are both acrylic resins, so that the monomer and the prepolymer have good compatibilities, and thus the mechanical property of the prepared resin composition is more excellent.

Preferably, the thermal initiator includes any one or combination of at least two of dibenzoyl peroxide, azodiisobutyronitrile, or di-tert-butyl peroxide.

Preferably, the photoinitiator includes any one or a combination of at least two of 2,4,6-trimethylbenzoyl-ethoxy-phenylphosphine oxide, 2,4,6-trimethylbenzoyl-diphenylphosphine oxide or bis(2,4,6-trimethylbenzoyl) phenylphosphine oxide.

Preferably, the fumed silica is the fumed silica after lipophilic treatment, wherein the fumed silica is fumed silica R711.

Preferably, a particle size of the fumed silica is 10~30 nm, and preferably, the particle size of the fumed silica is 12 nm, 14 nm, 16 nm, 18 nm, 20 nm, 22 nm, 24 nm, 26 nm, or 28 nm.

Preferably, the glass powder is the glass powder containing barium.

The glass powder includes the glass powder with a particle size of 0.6~0.8 µm and the glass powder with a particle size of 0.3~0.5 µm. Preferably, the particle size of the glass powder with the particle size of 0.6~0.8 µm is 0.62 µm, 0.64 µm, 0.66 µm, 0.68 µm, 0.7 µm, 0.72 µm, 0.74 µm, 0.76 µm, or 0.78 µm, and the particle size of the glass powder with the particle size of 0.3~0.5 µm is 0.32 µm, 0.34 µm, 0.36 µm, 0.38 µm, 0.4 µm, 0.42 µm, 0.44 µm, 0.46 µm, or 0.48 µm.

As a preferred technical solution of the present disclosure, the fumed silica with a particle size of 10~30nm together with the glass powders with the particle size of 0.6~0.8µm and the particle size of 0.3~0.5µm are adopted as fillers, which is more helpful to enhance the hardness, strength, and abrasion resistance of the resin composition.

Preferably, a mass ratio between the glass powder with the particle size of 0.6~0.8 µm and the glass powder with the particle size of 0.3~0.5 µm is (1~3): 1; and preferably, the mass ratio between the glass powder with the particle size of 0.6~0.8 µm and the glass powder with particle size of 0.3~0.5 µm is 1.2: 1, 1.4: 1, 1.6: 1, 1.8: 1, 2: 1, 2.2: 1, 2.4: 1, 2.6: 1, or 2.8: 1.

Preferably, the resin composition further includes any one or a combination of at least two of the catalyst, dispersant, antifoaming agent, colorant, or inhibitor.

Preferably, the content of the catalyst in the resin composition is 0.05~0.2 weight parts, preferably, 0.07 weight parts, 0.09 weight parts, 0.11 weight parts, 0.13 weight parts, 0.15 weight parts, 0.17 weight parts, or 0.19 weight parts.

Preferably, the catalyst includes dimethylaminoethyl methacrylate.

Preferably, the content of the dispersant in the resin composition is 0.01~5 weight parts, preferably 0.05 weight parts, 0.1 weight parts, 0.5 weight parts, 1 weight parts, 1.5 weight parts, 2 weight parts, 2.5 weight parts, 3 weight parts, 3.5 weight parts, 4 weight parts, or 4.5 weight parts.

Preferably, the dispersant includes any one or a combination of at least two of zinc stearate, sodium stearate, or stearic acid.

Preferably, the content of the antifoaming agent in the resin composition is 0.01~5 weight parts, preferably 0.05 weight parts, 0.1 weight parts, 0.5 weight parts, 1 weight parts, 1.5 weight parts, 2 weight parts, 2.5 weight parts, 3 weight parts, 3.5 weight parts, 4 weight parts, or 4.5 weight parts.

Preferably, the antifoaming agent includes any one or a combination of at least two of a polymer antifoaming agent, organosilicon antifoaming agent, or organosilicon/polymer composite antifoaming agent, wherein the organosilicon antifoaming agent includes an antifoaming agent BYK-067A.

Preferably, the content of the colorant in the resin composition is 0~5 weight parts and not equal to 0, preferably, 0.05 weight parts, 0.1 weight parts, 0.5 weight parts, 1 weight parts, 1.5 weight parts, 2 weight parts, 2.5 weight parts, 3 weight parts, 3.5 weight parts, 4 weight parts, or 4.5 weight parts.

Preferably, the colorant includes any one or a combination of at least two of titanium oxide, iron oxide yellow, iron oxide red, iron oxide black, or carbon black.

Preferably, the content of the inhibitor in the resin composition is 0.005~0.1 weight parts, preferably, 0.007 weight parts, 0.009 weight parts, 0.01 weight parts, 0.03 weight parts, 0.06 weight parts, or 0.09 weight parts.

Preferably, the inhibitor includes any one or a combination of at least two of 2,6-di-tert-butyl-p-cresol, tert-butyl-catechol, or p-phenol monobutyl ether.

In a second aspect, the present disclosure provides a preparation method of the resin composition as described in the first aspect, wherein the preparation method includes the following steps:
(1) mixing the monomer, photoinitiator, thermal initiator, optional dispersant, optional catalyst, optional antifoaming agent, and optional colorant, so as to obtain a mixed monomer;
(2) mixing the mixed monomer obtained in step (1) and the prepolymer, so as to obtain a mixed resin; and
(3) mixing the mixed resin obtained in step (2), the fumed silica, the and glass powder, so as to obtain the resin composition.

Preferably, a temperature of the mixing of the step (1)~step (3) is each 40~80°C independently, preferably, 45°C, 50°C, 55°C, 60°C, 65°C, 70°C, or 75°C, independently.

Preferably, a duration of the mixing of step (1)~step (3) is each 0.5~4h, independently, preferably, 0.8h, 1h, 1.5h, 2h, 2.5h, 3h, or 3.5h, independently.

In a third aspect, the present disclosure provides a temporary crown and bridge by 3D printing, wherein the preparation raw material of the temporary crown and bridge by 3D printing includes the resin composition as described in the first aspect.

The temporary crown and bridge by 3D printing provided by the present disclosure does not irritate the mucosa of oral tissues, which is a biocompatible material. Its water absorption value, dissolution value, and cytotoxicity are all qualified, which satisfies the industry standard requirement of YY0268-2008. The inorganic fumed silica and glass powder containing barium are added to the adopted resin composition, so that the temporary crown and bridge by 3D printing has better abrasion resistance than traditional materials and the PMMA materials.

In a fourth aspect, the present disclosure provides a preparation method of the temporary crown and bridge by 3D printing as described in the third aspect, wherein the preparation method includes: 3D printing and post-curing the resin composition as described in the first aspect, so as to obtain the temporary crown and bridge by 3D printing.

The 3D printing technology does not produce leftover materials, which greatly increases the utilization rate of materials, so as to greatly reduce the cost and also greatly reduce the operation time of the user, which needs a lower skill of the operator.

Preferably, the post-curing includes the photo curing and thermal curing.

Preferably, the wavelength of the photo curing is 355∼410nm, preferably, 360nm, 365nm, 370nm, 375nm, 380nm, 385nm, 390nm, 395nm, 400nm, or 405nm.

Preferably, the temperature of thermal curing is 30-100°C, preferably, 35°C, 40°C, 45°C, 50°C, 55°C, 60°C, 70°C, 80°C, or 90°C.

Preferably, a step of filtering the resin composition is further included before the 3D printing.

Preferably, the filtering is to pass the resin composition through a filter screen with an aperture of 40~100 mesh; preferably, the aperture of the filter screen is 50 mesh, 60 mesh, 70 mesh, 80 mesh, or 90 mesh.

Preferably, the steps of cleaning and drying are further included before the post-curing.

Preferably, the steps of sanding and polishing are further included after the post-curing.

As a preferred technical solution of the present disclosure, the preparation method includes: filtering the resin composition as described in the first aspect through the filter screen of 40~100 mesh; 3D printing, cleaning and drying; and UV curing under 355~410 nm and thermal curing under 30-100°C, and sanding and polishing, so as to obtain the temporary crown and bridge by 3D printing.

The preparation method of the crown and bridge resin provided by the present disclosure is to 3D print the resin composition as described in the first aspect. The molded crown and bridge molded is photo cured in a UV box with the heating function and the wavelength of 355~450 nm. The thermal curing is reacted at the same time to provide the material with a stronger physical property.

In a fifth aspect, the present disclosure provides use of the temporary crown and bridge of 3D printing as described in the third aspect as the oral restorative material or oral implant material.

Compared with the prior art, the present disclosure has the following beneficial effects.
(1) The photothermal-curing resin composition provided by the present disclosure includes the specific parts of the prepolymer, monomer, photoinitiator, thermal initiator, fumed silica, and glass powder. The system is added a specific number of the photoinitiator and thermal initiator, and the system is initiated by utilizing the photoinitiator and thermal initiator, so as to improve the completion rate of the curing reaction. Therefore, the curing is more fully, so that residual amount of the monomer in the finally obtained resin composition can meet the industry requirements. The specific parts of fumed silica and glass powder containing barium are further added to the system as the fillers, which enhances the hardness, strength, and abrasion resistance of the material, so that the obtained resin composition has the higher hardness and abrasion resistance after printed into the temporary crown and bridge by the 3D printer, which fully satisfies the requirements of the oral restorative material or oral implant material.
(2) Specifically, a viscosity of the resin composition of the obtained photothermal-curing resin by the present disclosure is 3076~3355 mPa·s; the monomer residue of the obtained temporary crown and bridge by 3D printing, prepared using the resin composition, is 0.43~0.95 mg/kg; the Vickers hardness 2.0 is 20.2~21.8 HV2/10; the water absorption value is 12~39 µg/mm³; the dissolution value is 2.6~3.2ug/mm³; the bending strength is 105∼121MPa, and the bending modulus of 2200~2760MPa.

### Detailed Description of Embodiments

The technical solutions of the present disclosure are further described below by specific embodiments. It should be clear to those skilled in the art that the described embodiments are merely to help to understand the present disclosure, and should not be regarded as specific limitations of the present disclosure.

### Example 1

A photothermal-curing resin composition was provided, and the resin composition included the following components by weight parts:

| | |
|---|---|
| a urethane dimethacrylate | 17 weight parts; |
| a bisphenol A-dimethacrylate glycidyl ester | 13 weight parts; |
| a triethylene glycol dimethacrylate | 26 weight parts; |
| a phenyl bis(2,4,6-trimethylbenzoyl)phosphine oxide | 0.35 weight parts; |
| a dibenzoyl peroxide | 0.2 weight parts; |
| a dimethylaminoethyl methacrylate | 0.15 weight parts; |
| a zinc stearate | 0.3 weight parts; |
| an antifoaming agent BYK-067A | 0.3 weight parts; |
| an iron oxide red | 0.03 weight parts; |
| an iron oxide yellow | 0.02 weight parts; |
| a titanium dioxide | 1.2 weight parts; |
| a fumed silica R711 | 8.5 weight parts; |
| a glass powder containing barium Nano Fine^{®} 0.4 | 11 weight parts; and |
| a glass powder containing barium Nano Fine^{®} 0.7 | 22.05 weight parts. |

The preparation method of the resin composition provided by the embodiment included the following steps:
(1) mixing the triethylene glycol dimethacrylate, phenyl bis(2,4,6-trimethylbenzoyl)phosphine oxide, dibenzoyl peroxide, antifoaming agent BYK-067A, zinc stearate, iron oxide red, iron oxide yellow, and titanium dioxide under 50°C for 2h to obtain a mixed monomer;
(2) mixing the mixed monomer obtained in step (1), urethane dimethacrylate, and bisphenol A-dimethacrylate glycidyl ester under 50°C for 2h to obtain a mixed resin; and
(3) mixing the mixed resin obtained in step (2), the fumed silica R711, the glass powder containing barium with a particle size of 0.4µm NanoFine^{®}0.4, and the glass powder containing barium with a particle size of 0.7µm NanoFine^{®}0.7 under 50°C for 2h to obtain the resin composition.

### Example 2

A photothermal-curing resin composition was provided, and the resin composition included the following components by weight parts:

| | |
|---|---|
| a urethane dimethacrylate | 17 weight parts; |
| a bisphenol A-dimethacrylate glycidyl ester | 13 weight parts; |
| a triethylene glycol dimethacrylate | 26 weight parts; |
| a (2,4,6-trimethylbenzoyl) diphenylphosphine oxide | 0.25 weight parts; |
| a dibenzoyl peroxide | 0.2 weight parts; |
| a dimethylaminoethyl methacrylate | 0.15 weight parts; |
| a zinc stearate | 0.3 weight parts; |
| an antifoaming agent BYK-067A | 0.3 weight parts; |
| an iron oxide red | 0.03 weight parts; |
| an iron oxide yellow | 0.02 weight parts; |
| a titanium dioxide | 1.2 weight parts; |
| a fumed silica R711 | 8.5 weight parts; |
| a glass powder containing barium Nano Fine^{®} 0.4 | 11 weight parts; and |
| a glass powder containing barium Nano Fine^{®} 0.7 | 22.5 weight parts. |

The preparation method of the resin composition provided by the embodiment was the same as Example 1.

### Example 3

A photothermal-curing resin composition was provided, and the resin composition included the following components by weight parts:

| | |
|---|---|
| a urethane dimethacrylate | 17 weight parts; |
| a bisphenol A-dimethacrylate glycidyl ester | 13 weight parts; |
| a hydroxyethyl methacrylate | 26 weight parts; |
| a (2,4,6-trimethylbenzoyl) diphenylphosphine oxide | 0.25 weight parts; |
| a dibenzoyl peroxide | 0.2 weight parts; |
| a dimethylaminoethyl methacrylate | 0.15 weight parts; |
| a zinc stearate | 0.3 weight parts; |
| an antifoaming agent BYK-067A | 0.3 weight parts; |
| an iron oxide red | 0.03 weight parts; |
| an iron oxide yellow | 0.02 weight parts; |
| a titanium dioxide | 1.2 weight parts; |
| a fumed silica R711 | 8.5 weight parts; |
| a glass powder containing barium Nano Fine^{®} 0.4 | 11 weight parts; and |
| a glass powder containing barium Nano Fine^{®} 0.7 | 22.5 weight parts. |

The preparation method of the resin composition provided by the embodiment was the same as Example 1.

### Example 4

A photothermal-curing resin composition was provided, which differed from Example 1 only in that the glass powder containing barium NanoFine^{®}0.4 was not added, the added amount of the glass powder containing barium NanoFine^{®}0.7 was 33.5 weight parts, and the other components, the dosages, and the preparation method were the same as that of Example 1.

### Example 5

A photothermal-curing resin composition was provided, which differed from Example 1 only in that the glass powder containing barium NanoFine^{®}0.7 was not added, the added amount of the glass powder containing barium NanoFine^{®}0.4 was 33.5 weight parts, and the other components, the dosages, and the preparation method were the same as that of Example 1.

### Comparative Example 1

A photothermal-curing resin composition was provided, which differed from Example 1 only in that the dibenzoyl peroxide was not added, the added amount of the phenyl bis(2,4,6-trimethylbenzoyl)phosphine oxide was 0.35 weight parts, and the other components, the dosages, and preparation method were the same as that of Example 1.

### Comparative Example 2

A photothermal-curing resin composition was provided, which differed from Example 1 only in that the fumed silica R711 was not added, the added amount of the glass powder containing barium NanoFine^{®}0.4 was 19.5 weight parts, and the other components, the dosages, and preparation methods were the same as that of Example 1.

### Comparative Example 3

A photothermal-curing resin composition was provided, which differed from Example 1 only in that the glass powder containing barium NanoFine^{®}0.4 and the glass powder containing barium NanoFine^{®}0.7 were not added, the added amount of fumed silica R711 was 41.55 weight parts, and the other components, the dosages, and preparation methods were the same as that of Example 1.

### Experimental Example 1

A temporary crown and bridge of 3D printing was provided, and the preparation method included: filtering the resin composition obtained from Example 1 through the filter screen of 40~100 mesh; 3D printing, then cleaning and drying; and curing for 20min under UV of 355~410 nm and thermal curing for 20min under 60°C, and sanding and polishing, so as to obtain the temporary crown and bridge by 3D printing.

### Experimental Examples 2~5

A temporary crown and bridge of 3D printing was provided, which differed from Experimental Example 1 only in that the resin composition obtained from Example 1 was replaced by the resin compositions obtained from Example 2~5 respectively, and all the other conditions and steps were the same as that of Experimental Example 1.

### Comparative Experimental Examples 1~3

A temporary crown and bridge by 3D printing was provided, which differed from Experimental Example 1 only in that the resin composition obtained from Example 1 was replaced by the resin compositions obtained from Comparative Example 1~3 respectively, and all the other conditions and steps were the same as that of Experimental Example 1.

### Performance Test:

(1) viscosity: tested according to "GB/T10248-2008 Viscosity Measurement Method";
(2) monomer residue: tested according to gas chromatography-mass spectrometry in "SN_T3342-2012 Determination of Residual Monomer Content in Acrylic Resin";
(3) Vickers hardness: tested according to the test method in Parts I of "GB/T4340.1-2009 Vickers Hardness";
(4) bending modulus and bending strength: tested according to "YY0710-2009 Dentistry-Polymer-based Crown and Bridge Materials"; and
(5) dissolution value and water absorption value: tested according to "YY0710-2009 Dentistry-Polymer-based Crown and Bridge Materials".

The resin compositions provided by Examples 1~5 and Comparative Examples 1~3 were tested according to the above test method (1), and the test results obtained are shown as Table 1.

Table 1 Viscosity of the resin compositions provided by Examples 1 ~ 5 and Comparative Examples 1 ~ 3

| | Example 1 | Example 2 | Example 3 | Example 4 | Example 5 | Comparative Example 1 | Comparative Example 2 | Comparative Example 3 |
|---|---|---|---|---|---|---|---|---|
| Viscosity (mPa·s) | 3241 | 3185 | 3076 | 3124 | 3355 | 3250 | 3026 | 5500 |

The temporary crown and bridges by 3D printing provided by Experimental Examples 1~5 and Comparative Experimental Examples 1~3 were tested according to the above test methods (2) ~ (5), and the test results obtained are shown as Table 2.

Table 2 Performance parameters of temporary crown and bridges by 3D printing provided by Experimental Examples 1~5 and Comparative Experimental Examples 1~3

| | monomer residue (mg/kg) | Vickers hardness 2.0(HV2/10) | water absorption value | dissolution value | bending strength | bending modulus |
|---|---|---|---|---|---|---|
| | | | (µg/mm³) | (µg/mm³) | (MPa) | (MPa) |
| Experimental Example 1 | 0.55 | 20.5 | 12 | 3.1 | 115 | 2350 |
| Experimental Example 2 | 0.43 | 20.2 | 15 | 2.8 | 121 | 2760 |
| Experimental Example 3 | 0.68 | 21.5 | 39 | 3.2 | 110 | 2270 |
| Experimental Example 4 | 0.89 | 20.8 | 21 | 2.7 | 108 | 2230 |
| Experimental Example 5 | 0.95 | 21.8 | 19 | 2.6 | 105 | 2200 |
| Comparative Experimental Example 2 | 35.8 | 19.5 | 45 | 8.3 | 98 | 2075 |
| Comparative Experimental Example 3 | 0.25 | 19.2 | 52 | 7.2 | 103 | 2100 |
| Comparative Experimental Example 4 | 835 | 21.9 | 152 | 12.8 | 54 | 1450 |

It can be seen from the data in Table 1 and Table 2 that the viscosities of the photothermal-curing resin compositions obtained by Examples 1~5 are 3076~3355 mPa·s, the monomer residues of the temporary crown and bridges by 3D printing obtained by Experimental Examples 1~5 are 0.43~0.95 mg/kg, the Vickers hardness 2.0 is 20.2~21.8 HV2/10, the water absorption values are 12~39 µg/mm³, the dissolution values are 2.6~3.2 µg/mm³, the bending strength is 105~121 MPa, and the bending modulus is 2200~2760 MPa.

Compared Experimental Example 1 with Comparative Experimental Example 1, it can be seen that the water absorption value of the temporary crown and bridge by 3D printing prepared by the resin compositions obtained by adding only the photoinitiator and not the thermal initiator is 45 µg/mm³, and the dissolution value is 8.3 µg/mm³, both of which are significantly increased compared to Experimental Example 1. Therefore, it does not satisfy the requirements of the "YY0710-2009 Dentistry-Polymer-based Crown and Bridge Materials" (the water absorption value and dissolution value need to be less than 40 µg/mm³ and 7.5 µg/mm³, respectively). The monomer residue of the obtained temporary crown and bridge by 3D printing in Comparative Experimental Example 1 is as high as 35.8 mg/kg.

Compared Experimental Example 1 and Comparative Experimental Example 2, it can be found that the resin composition obtained without the addition of fumed silica lacks a filling effect of nanoscale inorganic filler, which results in the water absorption value and the dissolution value of the temporary crown and bridge by 3D printing prepared thereof are higher, and the bending strength, the bending modulus, and the surface hardness are reduced.

Compared Experimental Example 1 with Comparative Experimental Example 3, it can be found that the fillers in the resin composition all use the fumed silica, and the glass powder containing barium are not added. The fumed silica is the nanoscale filler, and the specific surface area is much larger than that of the glass powder containing barium, so that the oil absorption of the resin composition significantly increases and the viscosity increases. Therefore, molding on the 3D printer is very difficult. The large oil absorption has a significant effect on resin crosslinking, which results in a very incomplete reaction of the organic components, a large amount of the monomer residue, a high water absorption value and dissolution value, and the bending strength and modulus property are very low.

Further compared Experimental Example 1 and Experimental Examples 4 ~ 5, it can be found that the monomer residue of the temporary crown and bridge by 3D printing, prepared by the resin composition obtained by the glass powder containing barium that only adds one type of particle size, is higher, and the bending strength and bending modulus are both reduced.

The applicant declares that the present disclosure illustrates a photothermal-curing resin composition and preparation method therefor and use thereof by the above embodiments.

## Claims

1. A photothermal-curing resin composition, wherein the resin composition comprises following components by weight parts:
| | |
|---|---|
| a prepolymer | 30 weight parts; |
| a monomer | 5~50 weight parts; |
| a photoinitiator | 0.01~5 weight parts; |
| a thermal initiator | 0.01~5 weight parts; |
| a fumed silica | 0.01~40 weight parts; and |
| a glass powder | 0.01~40 weight parts, |
wherein the prepolymer comprises any one or a combination of at least two of polyurethane acrylate, urethane dimethacrylate, bisphenol A-dimethacrylate glycidyl ester, ethoxybisphenol A dimethacrylate, or bisphenol A glycerol dimethacrylate, the monomer comprises any one or a combination of at least two of triethylene glycol dimethacrylate, hydroxyethyl methacrylate, or hydroxypropyl methacrylate, and the glass powder comprises glass powder with a particle size of 0.6~0.8 µm and glass powder with a particle size of 0.3~0.5 µm.

2. The resin composition according to claim 1, wherein the thermal initiator comprises any one or a combination of at least two of dibenzoyl peroxide, azodiisobutyronitrile, or di-tert-butyl peroxide.

3. The resin composition according to claim 1, wherein the photoinitiator comprises any one or a combination of at least two of 2,4,6-trimethylbenzoyl-ethoxy-phenylphosphine oxide, 2,4,6-trimethylbenzoyl-diphenylphosphine oxide, or bis(2,4,6-trimethylbenzoyl)phenylphosphine oxide.

4. The resin composition according to claim 1, wherein the fumed silica is fumed silica after lipophilic treatment.

5. The resin composition according to claim 1 or 6, wherein a particle size of the fumed silica is 10~30 nm.

6. The resin composition according to claim 1, wherein the glass powder is glass powder containing barium.

7. The resin composition according to claim 1 or 6, wherein a mass ratio between the glass powder with the particle size of 0.6~0.8 µm and the glass powder with the particle size of 0.3~0.5 µm is (1~3): 1.

8. The resin composition according to claim 1, wherein the resin composition further comprises any one or a combination of at least two of a catalyst, a dispersant, an antifoaming agent, a colorant, and an inhibitor,
preferably, a content of the catalyst in the resin composition is 0.05~0.2 weight parts;
preferably, the catalyst comprises dimethylaminoethyl methacrylate;
preferably, a content of the dispersant in the resin composition is 0.01 ~ 5 weight parts;
preferably, the dispersant comprises any one or a combination of at least two of zinc stearate, sodium stearate, and stearic acid;
preferably, a content of the antifoaming agent in the resin composition is 0.01~5 weight parts;
preferably, the antifoaming agent comprises any one or a combination of at least two of a polymer antifoaming agent, organosilicon antifoaming agent, or organosilicon/polymer composite antifoaming agent;
preferably, a content of the colorant in the resin composition is 0 ~ 5 weight parts and not equal to 0;
preferably, the colorant comprises any one or a combination of at least two of titanium oxide, iron oxide red, iron oxide yellow, iron oxide black, or carbon black;
preferably, a content of the inhibitor in the resin composition is 0.005 ~ 0.1 weight parts; and
preferably, the inhibitor comprises any one or a combination of at least two of 2,6-di-tert-butyl-p-cresol, tert-butyl-catechol, or p-phenol monobutyl ether.

9. A preparation method of the resin composition according to any one of claims 1~8, wherein the preparation method comprises following steps:
(1) mixing the monomer, the photoinitiator, the thermal initiator, an optional dispersant, an optional antifoaming agent, an optional catalyst, and an optional colorant, so as to obtain a mixed monomer;
(2) mixing the mixed monomer obtained in step (1) and the prepolymer, so as to obtain a mixed resin; and
(3) mixing the mixed resin obtained in step (2), the fumed silica, and the glass powder, so as to obtain the resin composition.

10. A temporary crown and bridge of 3D printing, wherein preparation raw materials of the temporary crown and bridge of 3D printing comprises the resin composition according to any one of claims 1~8 or the resin composition prepared by the preparation method according to claim 11.

11. A preparation method of the temporary crown and bridge of 3D printing according to claim 10, wherein the preparation method comprises: 3D printing and post-curing the resin composition according to any one of claims 1~8 or the resin composition prepared by the preparation method according to claim 11, so as to obtain the temporary crown and bridge of 3D printing.

12. The preparation method according to claim 11, wherein the post-curing comprises a combination of photo curing and thermal curing,
preferably, a wavelength of the photo curing is 355~410 nm; and
preferably, a temperature of the thermal curing is 30~100°C.

13. Use of the temporary crown and bridge of 3D printing according to claim 10 or the temporary crown and bridge of 3D printing prepared by the preparation method according to claim 11 or 12 as an oral restorative material or an oral implant material.

## Patentansprüche

1. Photothermisch härtbare Harzzusammensetzung, wobei die Harzzusammensetzung folgende Komponenten in Gewichtsteilen umfasst:
ein Präpolymer 30 Gewichtsteile;
ein Monomer 5~50 Gewichtsteile;
einen Photoinitiator0,01~5 Gewichtsteile;
einen thermischen Initiator 0,01~5 Gewichtsteile;
ein pyrogenes Siliciumoxid 0,01~40 Gewichtsteile; und
ein Glaspulver 0,01~40 Gewichtsteile,
wobei das Präpolymer ein beliebiges oder eine Kombination von mindestens zwei aus Polyurethanacrylat, Urethandimethacrylat, Bisphenol-A-Dimethacrylat-Glycidylester,
Ethoxybisphenol-A-Dimethacrylat oder Bisphenol-A-Glycerindimethacrylat umfasst, das Monomer ein beliebiges oder eine Kombination von mindestens zwei aus Triethylenglykoldimethacrylat, Hydroxyethylmethacrylat oder Hydroxypropylmethacrylat umfasst und das Glaspulver Glaspulver mit einer Partikelgröße von 0,6 bis 0,8 µm und Glaspulver mit einer Partikelgröße von 0,3~0,5 µm umfasst.

2. Harzzusammensetzung nach Anspruch 1, wobei der thermische Initiator ein beliebiges oder eine Kombination von mindestens zwei aus Dibenzoylperoxid, Azodiisobutyronitril oder Di-tert-butylperoxid umfasst.

3. Harzzusammensetzung nach Anspruch 1, wobei der Photoinitiator ein beliebiges oder eine Kombination von mindestens zwei aus 2,4,6-Trimethylbenzoyl-ethoxy-phenylphosphinoxid, 2,4,6-Trimethylbenzoyl-diphenylphosphinoxid oder Bis(2,4,6-trimethylbenzoyl)phenylphosphinoxid umfasst.

4. Harzzusammensetzung nach Anspruch 1, wobei das pyrogene Siliciumoxid pyrogenes Siliciumoxid nach lipophiler Behandlung ist.

5. Harzzusammensetzung nach Anspruch 1 oder 6, wobei eine Partikelgröße des pyrogenen Siliciumoxids 10~30 nm ist.

6. Harzzusammensetzung nach Anspruch 1, wobei das Glaspulver Glaspulver ist, das Barium enthält.

7. Harzzusammensetzung nach Anspruch 1 oder 6, wobei ein Massenverhältnis zwischen dem Glaspulver mit der Partikelgröße von 0,6~0,8 µm und dem Glaspulver mit der Partikelgröße von 0,3~0,5 µm (1~3): 1 ist.

8. Harzzusammensetzung nach Anspruch 1, wobei die Harzzusammensetzung ferner ein beliebiges oder eine Kombination von mindestens zwei aus einem Katalysator, einem Dispergiermittel, einem Antischaummittel, einem Farbmittel und einem Inhibitor umfasst,
ein Gehalt des Katalysators in der Harzzusammensetzung vorzugsweise 0,05~0,2 Gewichtsteile ist;
der Katalysator vorzugsweise Dimethylaminoethylmethacrylat umfasst;
ein Gehalt des Dispergiermittels in der Harzzusammensetzung vorzugsweise 0,01~5 Gewichtsteile ist;
das Dispergiermittel vorzugsweise ein beliebiges oder eine Kombination von mindestens zwei aus Zinkstearat, Natriumstearat und Stearinsäure umfasst;
ein Gehalt des Antischaummittels in der Harzzusammensetzung vorzugsweise 0,01~5 Gewichtsteile ist;
das Antischaummittel vorzugsweise ein beliebiges oder eine Kombination von mindestens zwei aus einem Polymer-Antischaummittel, einem Organosilicium-Antischaummittel oder einem Organosilicium/Polymer-Zusammensetzungs-Antischaummittel umfasst;
ein Gehalt des Farbmittels in der Harzzusammensetzung vorzugsweise 0~5 Gewichtsteile und nicht gleich 0 ist;
das Farbmittel vorzugsweise ein beliebiges oder eine Kombination von mindestens zwei aus Titanoxid, Eisenoxidrot, Eisenoxidgelb, Eisenoxidschwarz oder Industrieruß umfasst;
ein Gehalt des Inhibitors in der Harzzusammensetzung vorzugsweise 0,005~0,1 Gewichtsteile ist; und
der Inhibitor vorzugsweise ein beliebiges oder eine Kombination von mindestens zwei aus 2,6-Di-tert-butyl-p-kresol, tert-Butylcatechol oder p-Phenolmonobutylether umfasst.

9. Herstellungsverfahren der Harzzusammensetzung nach einem der Ansprüche 1 bis 8, wobei das Herstellungsverfahren folgende Schritte umfasst:
(1) Mischen des Monomers, des Photoinitiators, des thermischen Initiators, eines optionalen Dispergiermittels, eines optionalen Antischaummittels, eines optionalen Katalysators und eines optionalen Farbstoffs, um ein Monomergemisch zu erhalten;
(2) Mischen des in Schritt (1) erhaltenen Monomergemisches mit dem Präpolymer, um ein Harzgemisch zu erhalten; und
(3) Mischen des in Schritt (2) erhaltenen Harzgemisches mit dem pyrogenen Siliciumdioxid und dem Glaspulver, um die Harzzusammensetzung zu erhalten.

10. Provisorische Krone und Brücke aus 3D-Druck, wobei Herstellungsrohmaterialien der provisorischen Krone und Brücke aus 3D-Druck die Harzzusammensetzung nach einem der Ansprüche 1 bis 8 oder die Harzzusammensetzung, die durch das Herstellungsverfahren nach Anspruch 11 hergestellt wird, umfassen.

11. Herstellungsverfahren einer provisorischen Krone und Brücke aus 3D-Druck nach Anspruch 10, wobei das Herstellungsverfahren Folgendes umfasst: 3D-Druck und Nachhärten der Harzzusammensetzung nach einem der Ansprüche 1 bis 8 oder der Harzzusammensetzung, die durch das Herstellungsverfahren nach Anspruch 11 hergestellt wird, um die provisorische Krone und Brücke aus 3D-Druck zu erhalten.

12. Herstellungsverfahren nach Anspruch 11, wobei das Nachhärten eine Kombination aus Photohärten und thermischem Härten umfasst, eine Wellenlänge des Photohärtens vorzugsweise 355-410 nm ist; und eine Temperatur des thermischen Härtens 30 bis 100 °C ist.

13. Verwendung der provisorischen Krone und Brücke aus 3D-Druck nach Anspruch 10 oder der provisorischen Krone und Brücke aus 3D-Druck, die durch das Herstellungsverfahren nach Anspruch 11 oder 12 hergestellt werden, als ein orales Restaurationsmaterial oder ein orales Implantatmaterial.

## Revendications

1. Composition de résine à durcissement photothermique, dans laquelle la composition de réseine comprend les composants suivants en parties en poids :
un prépolymère 30 parties en poids ;
un monomère de 5 à 50 parties en poids ;
un photo-initiateur de 0,01 à 5 parties en poids ;
un initiateur thermique de 0,01 à 5 parties en poids ;
de la silice sublimée de 0,01 à 40 parties en poids ; et
de la poudre de verre de 0,01 à 40 parties en poids,
dans laquelle le prépolymère comprend l'un quelconque ou une combinaison d'au moins deux parmi de l'acrylate de polyuréthane, du diméthacrylate d'uréthane, un ester glycidylique de diméthacrylate de bisphénol A, du diméthacrylate d'éthoxybisphénol A ou du diméthacrylate de glycérol de bisphénol A ; le monomère comprend l'un quelconque ou une combinaison d'au moins deux parmi du diméthacrylate de triéthylène glycol, du méthacrylate d' hydroxyéthyle ou du méthacrylate d'hydroxypropyle ; et la poudre de verre comprend une poudre de verre présentant une taille de particules de 0,6 à 0,8 µm et une poudre de verre présentant une taille de particules de 0,3 à 0,5 µm.

2. Composition de résine selon la revendication 1, dans laquelle l'initiateur thermique comprend l'un quelconque ou une combinaison d'au moins deux parmi du peroxyde de dibenzoyle, de l'azodiisobutyronitrile ou du peroxyde de di-tert-butyle.

3. Composition de résine selon la revendication 1, dans laquelle le photo-initiateur comprend l'un quelconque ou une combinaison d'au moins deux parmi de l'oxyde de 2,4,6-triméthylbenzoyl-éthoxy-phénylphosphine, de l'oxyde de 2,4,6-triméthylbenzoyl-diphénylphosphine ou de l'oxyde de bis(2,4,6-triméthylbenzoyl)phénylphosphine.

4. Composition de résine selon la revendication 1, dans laquelle la silice sublimée est de la silice sublimée après traitement lipophile.

5. Composition de résine selon la revendication 1 ou 6, dans laquelle une taille de particules de la silice sublimée est de 10 à 30 nm.

6. Composition de résine selon la revendication 1, dans laquelle la poudre de verre est de la poudre de verre contenant du baryum.

7. Composition de résine selon la revendication 1 ou 6, dans laquelle un rapport de masse entre la poudre de verre présentant une taille de particules de 0,6 à 0,8 µm et la poudre de verre présentant une taille de particules de 0,3 à 0,5 µm est de (1 à 3) : 1.

8. Composition de résine selon la revendication 1, dans laquelle la composition de résine comprend en outre l'un quelconque ou une combinaison d'au moins deux parmi un catalyseur, un dispersant, un agent antimoussant, un colorant et un inhibiteur,
de préférence, une teneur en catalyseur dans la composition de résine est de 0,05 à 0,2 parties en poids ;
de préférence, le catalyseur comprend du méthacrylate de diméthylaminoéthyle ;
de préférence, une teneur en dispersant dans la composition de résine est de 0,01 à 5 parties en poids ;
de préférence, le dispersant comprend l'un quelconque ou une combinaison d'au moins deux parmi du stéarate de zinc, du stéarate de sodium et de l'acide stéarique ;
de préférence, une teneur en agent antimoussant dans la composition de résine est de 0,01 à 5 parties en poids ;
de préférence, l'agent antimoussant comprend l'un quelconque ou une combinaison d'au moins deux parmi un agent antimoussant polymère, un agent antimoussant organosilicié ou un agent antimoussant composite organosilicié/polymère ;
de préférence, une teneur en colorant dans la composition de résine est de 0 à 5 parties en poids et non égale à 0 ;
de préférence, le colorant comprend l'un quelconque ou une combinaison d'au moins deux parmi de l'oxyde de titane, de l'oxyde de fer rouge, de l'oxyde de fer jaune, de l'oxyde de fer noir ou du noir de carbone ;
de préférence, une teneur en inhibiteur dans la composition de résine est de 0,005 à 0,1 partie en poids ; et
de préférence, l'inhibiteur comprend l'un quelconque ou une combinaison d'au moins deux parmi du 2,6-di-tert-butyl-p-crésol, du tert-butyl-catéchol ou de l'éther monobutylique de p-phénol.

9. Procédé de préparation de la composition de résine selon l'une quelconque des revendications 1 à 8, dans lequel le procédé de préparation comprend les étapes suivantes :
(1) le mélange du monomère, du photo-initiateur, de l'initiateur thermique, d'un dispersant facultatif, d'un agent antimoussant facultatif, d'un catalyseur facultatif et d'un colorant facultatif, de sorte à obtenir un monomère mixte ;
(2) le mélange du monomère mixte obtenu à l'étape (1) et du prépolymère, de sorte à obtenir une résine mixte ; et
(3) le mélange de la résine mixte obtenue à l'étape (2), de la silice sublimée et de la poudre de verre, de sorte à obtenir la composition de résine.

10. Couronne et bridge provisoires obtenus par impression 3D, dans lesquels les matières premières de préparation de la couronne et du bridge provisoires obtenus par impression 3D comprennent la composition de résine selon l'une quelconque des revendications 1 à 8 ou la composition de résine préparée au moyen du procédé de préparation selon la revendication 11.

11. Procédé de préparation de la couronne et du bridge provisoires obtenus par impression 3D selon la revendication 10, dans lequel le procédé de préparation comprend : l'impression 3D et le post-durcissement de la composition de résine selon l'une quelconque des revendications 1 à 8 ou de la composition de résine préparée au moyen du procédé de préparation selon la revendication 11, de sorte à obtenir la couronne et le bridge provisoires par impression 3D.

12. Procédé de préparation selon la revendication 11, dans lequel le post-durcissement comprend une combinaison de photo-durcissement et de durcissement thermique,
de préférence, une longueur d'onde du photo-durcissement est de 355 à 410 nm ; et
de préférence, une température du durcissement thermique est de 30 à 100 °C.

13. Utilisation de la couronne et du bridge provisoires obtenus par impression 3D selon la revendication 10 ou de la couronne et du bridge provisoires obtenus par impression 3D préparés au moyen du procédé de préparation selon la revendication 11 ou 12 comme matériau de restauration orale ou matériau d'implant oral.
